# EUROPÄISCHE PATENTSCHRIFT

(11) **EP 1 016 858 B1**
(45) Veröffentlichungstag und Bekanntmachung des Hinweises auf die Patenterteilung: **14.11.2001**
(21) Anmeldenummer: 00106666.1
(22) Anmeldetag: 19.12.1996
(51) Int. Cl.: G01J 5/04, G01N 33/20

(54) **Verfahren und Vorrichtung zur Bestimmung von aus einer Schmelze ausgehenden elektromagnetischen Wellen**
Method and device for measuring electromagnetic waves emanating from a melt
Procédé et appareil pour la mesure d'ondes electromagnetiques émanant d'une matière fondue

(30) Priorität: 20.12.1995 AT 208195
(43) Veröffentlichungstag der Anmeldung: 05.07.2000
(62) Teilanmeldung aus: 96942176.7
(73) Patentinhaber: VOEST-ALPINE INDUSTRIEANLAGENBAU GMBH, 4020 Linz (AT)
(72) Erfinder: Fritz, Ernst Dipl.-Ing., 4020 Linz (AT); Ramaseder, Norbert, Dipl.-Ing. Dr., 4020 Linz (AT)
(74) Vertreter: Kopecky, Helmut, Dipl.-Ing.

(56) Entgegenhaltungen:
- EP-A- 0 162 949
- EP-A- 0 362 577
- DE-A- 4 025 909
- FR-A- 2 514 894
- US-A- 3 161 499
- US-A- 4 619 533

## Beschreibung

Die Erfindung betrifft ein Verfahren zur Bestimmung von aus dem Inneren einer Schmelze, insbesondere einer Metallschmelze, ausgehenden elektromagnetischen Wellen, insbesondere im Bereich des sichtbaren Lichts und des angrenzenden UV-Bereiches und Infrarotbereiches, wobei in der Schmelze durch Einblasen von Gas ein gasgefüllter Hohlraum gebildet wird und von der Schmelze emittierende elektromagnetische Wellen durch das eingeblasene Gas hindurch beobachtet und ausgewertet werden, indem die elektromagnetischen Wellen über ein optisches System einem Detektor zur Bestimmung der Temperatur und/oder der chemischen Zusammensetzung zugeleitet werden, sowie eine Einrichtung zur Durchführung des Verfahrens.

Bei der Stahlerzeugung in einem Konverter oder einem anderen metallurgischen Reaktor durch Frischen von Roheisen bzw. beim Behandeln anderer Schmelzen in einem solchen metallurgischen Gefäß gibt es seit jeher das Bestreben, während des laufenden Behandlungsvorganges möglichst kontinuierlich und schnell über Temperaturwerte der Schmelze und/oder über eine Schmelzenanalyse zu verfügen, um den Behandlungsprozeß möglichst kurz halten zu können und der beabsichtigten Zielanalyse möglichst nahe zu kommen. Schnelligkeit ist insbesondere deswegen erforderlich, weil die chemischen Umsetzreaktionen mit großer Geschwindigkeit ablaufen und die Gefahr besteht, nicht mehr rechtzeitig in den Frischprozeß bzw. Behandlungsprozeß eingreifen zu können. Die extrem rauhen Betriebsbedingungen in solchen Anlagen kommen dieser Aufgabenstellung nicht entgegen. Bei der Stahlerzeugung in einem metallurgischen Reaktor (Konverter, Elektroofen etc.), bei sekundärmetallurgischer Behandlung von Stahlschmelzen bzw. bei anderen nichteisenmetallischen Schmelzen (z.B. Cu, Ni, Al) ist es weiters von Vorteil, die Temperatur bzw. Analyse der Schmelze nach jedem Behandlungsschritt zu kennen.

Zur Lösung dieser Problematik ist beispielsweise versucht worden, aus der spektralen Analyse der Konverterflamme oder aus ihrer Absorptionswirkung gegenüber monochromatischem Licht bestimmter Wellenlänge einen Hinweis für den richtigen Zeitpunkt der Beendigung des Frischprozesses zu erhalten. Die stark wechselnden Blasbedingungen und die schäumende Schlacke auf dem Schmelzenbad sowie der hohe Staubgehalt im Abgas erlauben jedoch keinen hinreichend genauen Rückschluß auf Badtemperatur und Schmelzenanalyse.

Weiters ist zur Temperaturmessung vorgeschlagen worden (DE-B - 14 08 873), in die feuerfeste Zustellung des Konverters gekapselte Thermoelemente einzusetzen, die in den Konverterinnenraum vorstehen und in Arbeitsstellung des Konverters unter dem Badspiegel der zu frischenden Schmelze liegen. Die Haltbarkeit dieser Thermoelemente war jedoch unzureichend; die notwendigerweise starke Kühlung der Meßeinrichtung beeinträchtigt zudem die Meßergebnisse.

Weiters ist es bekannt, die Temperatur einer Schmelze zu einem bestimmten Zeitpunkt mittels einer in die Schmelze eintauchenden Lanze zu bestimmen. Dieses Verfahren ist bei Anwendung der Stahlherstellung in einem Konverter nachteilig, denn hierzu muß der Konverter gekippt und wieder aufgerichtet werden, was einen Temperaturverlust des Stahlbades bis zu 40°C verursacht. Das Verfahren ist weiters zeitintensiv, denn zunächst muß vor dem Kippen des Konverters die Blaslanze ausgefahren werden, und es muß nach der Durchführung der Messung der Konverter wieder aufgerichtet werden, worauf erst - falls notwendig - die Blaslanze eingefahren und weiter geblasen werden kann. Weitere Nachteile sind, daß der Meßpunkt in der Schmelze nur willkürlich gewählt werden kann, also kaum reproduzierbar ist. Weiters ist auch die Eintauchtiefe der Sonde nicht genau feststellbar und ebenfalls kaum reproduzierbar.

Wesentlich komplizierter gestaltet sich noch die Bestimmung einer chemischen Analyse der Schmelze. Hierfür ist es bekannt, Proben zu entnehmen, u.zw. mit Hilfe von in die Schmelze eintauchenden Lanzen. Für die Stahlerzeugung im Konverter ergeben sich hierdurch Nachteile, da eine solche Probenahme ebenfalls zeitintensiv ist - der Konverter muß in diesem Fall ebenfalls gekippt werden (ausgenommen bei senkrechter Sublanzenmessung) - und die Probe muß ins Labor gebracht werden.

Es ist bekannt, bei der Herstellung von Stahl im Konverter eine Kohlenstoff-Schnellbestimmung durchzuführen, u.zw. durch Messung des Haltepunktes der Temperatur und des C-Gehaltes. Hierdurch gelingt es jedoch nur, das C-Äquivalent zu erfassen, so daß zur Berechnung des tatsächlichen Kohlenstoffgehaltes einige der in der Schmelze vorhandenen Begleitelemente berücksichtigt werden müssen.

Weiters ist es bekannt, mit Hilfe von Sublanzen Kohlenstoff- und Sauerstoffaktivitätsbestimmungen sowie Probenahmen und Temperaturmessungen in einem Konverter durchzuführen. Dies ist jedoch insoferne nachteilig, als die Sublanzeneinrichtungen selbst (und auch die Proben) sehr teuer sind, einem unverhältnismäßig großen Verschleiß unterliegen und nur bei flüssigen Schlacken gegen Ende des Blasprozesses eingesetzt werden können.

Aus der EP-B - 0 162 949 ist ein Verfahren zur Beobachtung der Schlackenbildung in einem Blasstahlkonverter bekannt, bei dem die im Konverterraum von der Schlackenoberfläche emittierte Lichtstrahlung herangezogen wird. Das Licht wird hierbei photoelektrisch in Signale umgewandelt und verarbeitet, wobei Veränderungen der Signale als Kriterium der Schaumschlackenbildung gewertet werden. Die in der Seitenwand des Konverters eingesetzten Rezeptoren befinden sich oberhalb des Schlacken-/Schmelzenbades und sind für die Messung der Schmelzenbadtemperatur und der Schmelzenzusammensetzung nicht geeignet.

Aus der US-A - 4,830,601 ist ein Verfahren und die Vorrichtung zur spektralanalytischen Auswertung des emittierten Lichts aus dem Zentrum einer Brennerflamme bekannt. Dabei wird die Zufuhr von Brennstoff und Verbrennungsluft anhand des Lichtspektrums überprüft. Über Glasfaserleitungen wird emittiertes Licht einer Auswerteelektronik zugeführt und die Verbrennungsluft- bzw. Brennstoffzufuhr entsprechend der ermittelten Gasanalyse geregelt.

Eine ähnliche Anordnung zur Temperaturmessung bei einem Verfahren zur Reduktionsgaserzeugung in einem Hochtemperaturreaktor bei erhöhtem Betriebsdruck ist der DE-A - 40 25 909 zu entnehmen.

Aus der EP-A - 0 215 483 ist es bekannt, die chemische Zusammensetzung des Eisens zu eruieren, indem Sauerstoff oder ein Sauerstoff enthaltendes Gas von oben auf die Oberfläche von geschmolzenem Eisen geblasen wird, wobei von der Schmelzenoberfläche ausgehende Strahlen in einem Spektrometer zur Bestimmung der chemischen Zusammensetzung des Eisens detektiert werden.

Aus der US-A - 4,619,533 und der EP-A - 0 362 577 sind Verfahren der eingangs beschriebenen Art bekannt, wobei im ersteren Fall von der Metallschmelze ausgehende Strahlung über einen Lichtwellenleiter einem Detektor zugeleitet wird. Gemäß der EP-A-0 362 577 wird Laserlicht auf die Metalloberfläche fokussiert und hierbei ein Plasma erzeugt. Das von der Metalloberfläche emittierte Plasmalicht wird über ein Linsensystem und einen Lichtwellenleiter einem Spektrometer zur Elementanalyse zugeführt. Das Linsensystem weist verstellbare Linsen auf. Die Linsen werden so eingestellt, daß das Intensitätsverhältnis zweier Eisenlinien, u.zw. die Intensität einer Atomlinie und die Intensität einer Ionenlinie minimal ist.

Das Einblasen von Gas zur Bildung eines gasgefüllten Hohlraumes erfolgt bei einem Verfahren der eingangs beschriebenen Art, d.h. bei Erfassen von aus dem Inneren einer Schmelze ausgehenden elektromagnetischen Wellen, vorteilhaft über eine Wandöffnung eines die Metallschmelze aufnehmenden metallurgischen Gefäßes, wobei diese Öffnung unterhalb des Normalbadspiegels liegen muß. Im Übergangsbereich dieser Öffnung des metallurgischen Gefäßes zur Schmelze hin, d.h. im Randbereich dieser Öffnung, gehen selbst bei sehr kleiner Öffnung Reflexionen der von der Schmelze emittierenden elektromagnetischen Wellen aus, die zu Meßwertverfälschungen führen. Kommt es zu einer pilzförmigen Ansatzbildung aus erstarrter Schmelze infolge eingeblasenen Gases, stellt der Ansatz, der die Form eines den Randbereich der Öffnung über ihren ganzen Umfang umgebenden und in Richtung Schmelze gerichteten Wulstes aufweist, trotz seiner Schutzfunktion für die Öffnung einen Störfaktor dar und verändert stets seine Größe und Lage, wodurch Strahlung, die von der Oberfläche des Ansatzes oder vom Übergangsbereich des Ansatzes zur Schmelze ausgeht, das Meßergebnis verfälscht. Es hat sich gezeigt, daß eine genaue Messung nur dann durchgeführt werden kann, wenn ausschließlich Strahlung von der Schmelzenoberfläche empfangen und dem Detektor zugeleitet wird. Reflexionen vom Randbereich der Öffnung bzw. vom Ansatz wirken stark störend, d.h. meßwertverfälschend, ohne daß dies durch andere Anzeichen bemerkbar ist.

Die Erfindung bezweckt die Vermeidung dieser Nachteile und Schwierigkeiten und stellt sich die Aufgabe, ein Verfahren der eingangs beschriebenen Art sowie eine Einrichtung zur Durchführung des Verfahrens zu schaffen, welche die Ermittlung gewünschter Meßwerte einer Schmelze (wie z.B. Stahl, rostfreier Stahl, Ferrolegierungen und Schmelzen von Nichteisenmetallen) praktisch ohne Zeitverzögerung auf einfache Art und Weise und insbesondere kontinuierlich sowie auch bei viskosen bis trockenen Schlacken ermöglichen. Hierbei sollen Meßwertverfälschungen, die durch das Meßverfahren selbst und die durch den rauhen Hüttenwerksbetrieb bedingt sind, zuverlässig vermieden werden können, wobei Meßwertverfälschungen auch bei sehr klein gehaltenem Hohlraum in der Schmelze ausgeschlossen werden sollen.

Diese Aufgabe wird erfindungsgemäß dadurch gelöst, daß schräg zur optischen Achse des optischen Systems gerichtete und vom Randbereich des Hohlraumes ausgehende elektromagnetische Wellen von einer Detektierung ausgeschlossen werden, indem die emittierenden elektromagnetischen Wellen von schräg zur optischen Achse des optischen Systems gerichteten und außerhalb eines von der optischen Achse des optischen Systems gezogenen Grenzradius vorhandenen elektromagnetischen Wellen befreit werden, indem diese elektromagnetischen Wellen in einer Wellenstreueinrichtung des optischem Systems, wie einem Zerstreuungs- und Sammellinsensystem, von der optischen Achse des optischen Systems weggebrochen werden und nur etwa parallel zur optischen Achse des optischen Systems gerichtete elektromagnetische Wellen zu einem dem optischen System nachgeordneten Detektor gelangen.

Vorzugsweise wird das optische System unter Ausrichtung seiner optischen Achse gegenüber dem Hohlraum bewegt, u.zw. so lange bewegt, bis die Intensität der emittierenden elektromagnetischen Wellen bei der Auswertung derselben ein Maximum ergibt.

Gemäß einer bevorzugten Ausführungsform ist der Wellenstreueinrichtung eine Wellenbündeleinrichtung, wie eine Sammellinse oder ein Sammellinsensystem, nachgeordnet und werden die etwa parallel zur optischen Achse des optischen Systems gerichteten elektromagnetischen Wellen von der Wellenbündeleinrichtung fokussiert und dem Detektor direkt oder über einen Lichtwellenleiter zugeleitet, die schrägen und außerhalb eines Grenzradius vorhandenen Wellen werden jedoch von dieser Fokussierung nicht mitumfaßt.

Eine weitere bevorzugte Ausführungsform ist dadurch gekennzeichnet, daß sowohl die Wellenstreueinrichtung als auch die nachgeordnete Strahlenbündeleinrichtung unter Ausrichtung ihrer optischen Achse gegenüber dem Hohlraum bewegt werden, bis die Intensität der emittierenden elektromagnetischen Wellen bei der Auswertung derselben ein Maximum ergibt. Hierdurch gelingt es, auch bei einer besonders starken und/oder bei einer stark einseitigen Ansatzbildung, d.h. bei Schmelzen mit besonders starker Neigung zur Ansatzbildung oder bei Hohlräumen in der Schmelze mit kleinen Durchmessern, noch optimale Meßergebnisse zu erhalten.

Zur Durchführung einer Schmelzenanalyse wird zweckmäßig über den gasgefüllten Hohlraum der Schmelze Energie zugeführt und durch die zugeführte Energie ein Teil der Schmelze verdampft, wobei vorteilhaft das eingeblasene Gas mit der Schmelze eine chemische Reaktion eingeht, die zum Verdampfen eines Teiles der Schmelze führt.

Zum Schutz des Meßvorganges wird zweckmäßig das zur Bildung des gasgefüllten Hohlraumes eingeblasene Gas an der Eintrittsstelle in die Schmelze von einem Gasmantel oder mehreren Gasmänteln, der bzw. die ein kohlenwasserstoffhältiges Schutzmedium, vorzugsweise gemischt mit Inertgas, enthält bzw. enthalten, umgeben. Hierdurch kommt es zur Ausbildung eines die Gaszuführung sichernden Ansatzes an erstarrter Schmelze, der auch eine wesentliche Schonung der für die Durchführung der Messung erforderlichen Einrichtung sowie lange Standzeiten derselben bewirkt.

Eine Vereinfachung und Beschleunigung des Verfahrens ergibt sich, wenn die Bestimmung der Temperatur bzw. chemischen Analyse der Schmelze kombiniert wird mit vorgerechneten bzw. gemessenen Parametern, wie einer Kohlenstoffbilanz der Abgasanalyse oder einer groben Berechnung der Analyse der Schmelze zum Zeitpunkt der Messung, und weiters, wenn nur der Gehalt einzelner Elemente der Schmelze, wie z.B. bei Eisenschmelzen der Mn-, Cr-, C-Gehalt etc., ermittelt wird und daraus der Gehalt der anderen in der Schmelze und auch Schlackenschmelze vorhandenen Elemente bzw. Verbindungen berechnet wird.

Die Genauigkeit des erfindungsgemäßen Verfahrens läßt sich dadurch steigern, daß während der Messung im Hohlraum bzw. knapp davor durch Einleiten eines Gasgemisches eine Temperatur eingestellt wird, die möglichst nahe an der Ist-Temperatur der Schmelze liegt.

Vorzugsweise wird mit einem Gas oder mit mehreren verschiedenen Gasen, die in die Schmelze eingeleitet werden, die chemische Analyse der Schmelze gezielt verändert und die Schmelze bzw. Schmelze und Schlacke durchmischt.

Nach einer bevorzugten Ausführungsform wird der gasgefüllte Hohlraum an der Schmelzenoberfläche gebildet, indem z.B. eine Gaszuführungsleitung mit Optik, Lichtwellenleiter, Detektor etc. in die Schmelze eintaucht.

Eine Einrichtung zur Durchführung des Verfahrens mit
- einem eine Schmelze aufnehmenden Gefäß,
- einer zu einer Öffnung des Gefäßes rührenden Gaszuführungsleitung mit einer gegen Öffnung und damit gegen die Schmelze gerichteten Gasaustrittsöffnung,
- einem optischen System zur Beobachtung der Gasaustrittsöffnung,
- einem Detektor zur Registrierung von aus der Schmelze emittierenden elektromagnetischen Wellen und
- gegebenenfalls mit einem die elektromagnetischen Wellen zum Detektor rührenden Wellenleiter,
ist gekennzeichnet durch
- eine optische Wellenstreueinrichtung, wie ein Zerstreuungs-Sammellinsensystem, wobei vorzugsweise
- eine gegenüber dem metallurgischen Gefäß bewegbare, vorzugsweise schwenkbare, Anordnung des optischen Systems vorgesehen ist.

Eine bevorzugte Ausführungsform ist gekennzeichnet durch
- eine der Wellenstreueinrichtung nachgeordnete Wellenbündeleinrichtung, wie eine Sammellinse oder ein nachgeordnetes Sammellinsensystem, und
- einen im Fokussierungsbereich der Wellenbündeleinrichtung liegenden Detektor oder einen dort angeordneten und zu einem Detektor führenden Lichtwellenleiter.

Vorteilhaft ist ein Schutzrohr für das optische System mit einer Gasspülung, insbesondere einer das Linsensystem an der Frontseite reinigenden Gasspülung, vorgesehen. Dies ist insbesondere dann erforderlich, falls durch die Gaszuführungsleitung Feststoffe, wie beispielsweise Schlackenbildner, Stäube, insbesondere Kohlenstaub, etc., zwischen den Meßzeitpunkten in die Schmelze eingeblasen werden.

Eine weitere bevorzugte Ausführungsform ist dadurch gekennzeichnet, daß die Wellenstreueinrichtung gegenüber der gegen die Schmelze gerichteten Gasaustrittsöffnung schwenkbar ist, wobei der Schnittpunkt der optischen Achse der Wellenstreueinrichtung mit der Querschnittsfläche der Gasaustrittsöffnung innerhalb deren Querschnittsfläche verstellbar ist.

Zweckmäßig sind sowohl die Wellenstreueinrichtung als auch die Wellenbündeleinrichtung schwenkbar gelagert, wobei vorteilhaft die schwenkbare Lagerung als kardanische Lagerung ausgebildet ist.

Entweder ist im Fokussierungsbereich der Wellenbündeleinrichtung ein Einlaß eines Lichtwellenleiters angeordnet oder ist im Fokussierungsbereich der Wellenbündeleinrichtung der Detektor angeordnet.

Eine zweckmäßige Ausführungsform ist dadurch gekennzeichnet, daß das Ende der Gaszuführungsleitung als Zwei- oder Mehrrohrdüse ausgebildet ist, deren Mantelringraum bzw. -räume an eine ein Kohlenwasserstoffgas zuführende Leitung anschließbar ist (sind). Hierdurch kommt es zur Ausbildung eines Ansatzes von erstarrter Schmelze, der die Gaseintrittsöffnung umgibt, so daß die Mehrrohrdüse gut geschützt am Gefäß, d.h. in der Ausmauerung des Gefäßes angeordnet ist.

Vorzugsweise ist das Ende der Gaszuführungsleitung von einer Mehrkanaldüse gebildet, deren Düsenöffnungen an eine oder mehrere Zuführungsleitungen für Kohlenwasserstoff, Kohlenmonoxid, Kohlendioxid, Inertgas, Dampf, Öl oder Wasser bzw. Mischungen davon anschließbar sind. Hierbei gelingt es, die Manteldüsenhaltbarkeit und Genauigkeit der Messung durch Einstellung der Menge und/oder Zusammensetzung der durch die Ringspalte eingeleiteten Gase oder Flüssigkeiten während des Durchführens des Meßverfahrens und generell zu optimieren.

Gemäß einer bevorzugten Ausführungsform ist, wie aus der EP-A - 0 362 577 an sich bekannt, eine gegen die Gasaustrittsöffnung der Gaszuführungsleitung gerichtete Laserstrahleinrichtung vorgesehen, wobei zweckmäßig der Laserstrahleneinrichtung eine Fokussiereinrichtung zugeordnet ist.

Vorzugsweise ist eine in die Schmelze eintauchende Gaszuführungsleitung mit Wellenstreueinrichtung vorgesehen.

Ein Verfahren zum Betrieb einer erfindungsgemäßen Einrichtung ist dadurch gekennzeichnet, daß zum Schutz des in das Gefäß mündenden Teiles der Einrichtung die Zufuhr des Schutzmediums geregelt wird, indem mit zunehmendem Angriff der Schmelze, d.h. mit steigender Temperatur bzw. Überhitzung der Schmelze, die Zufuhr von kohlenwasserstoffhältigem Schutzmedium kontinuierlich oder stufenweise verstärkt wird.

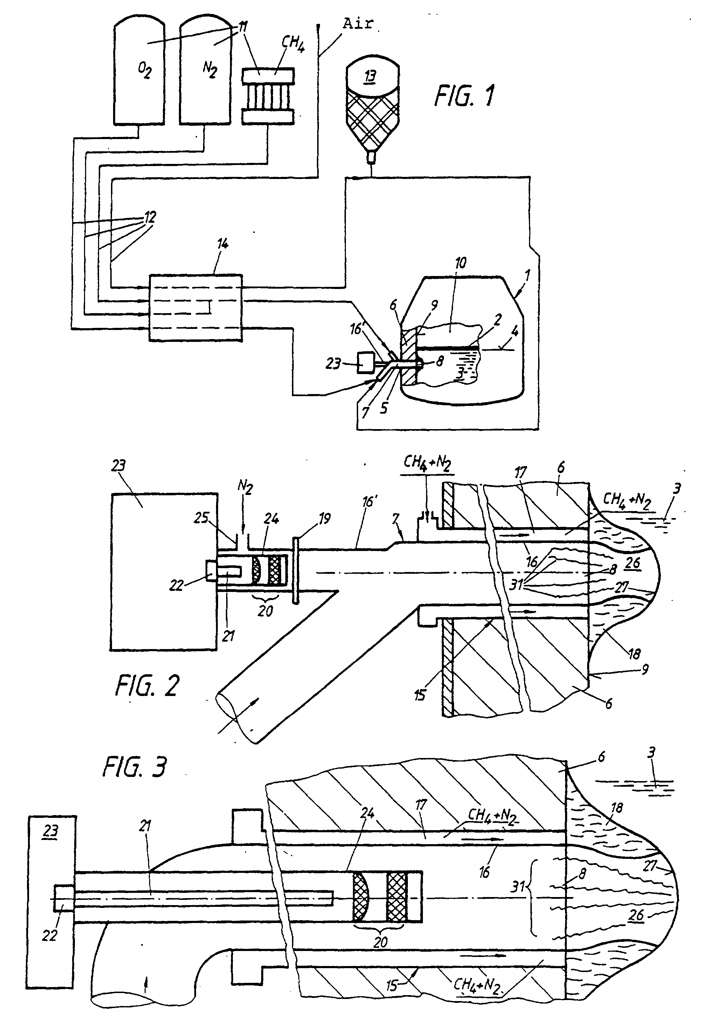

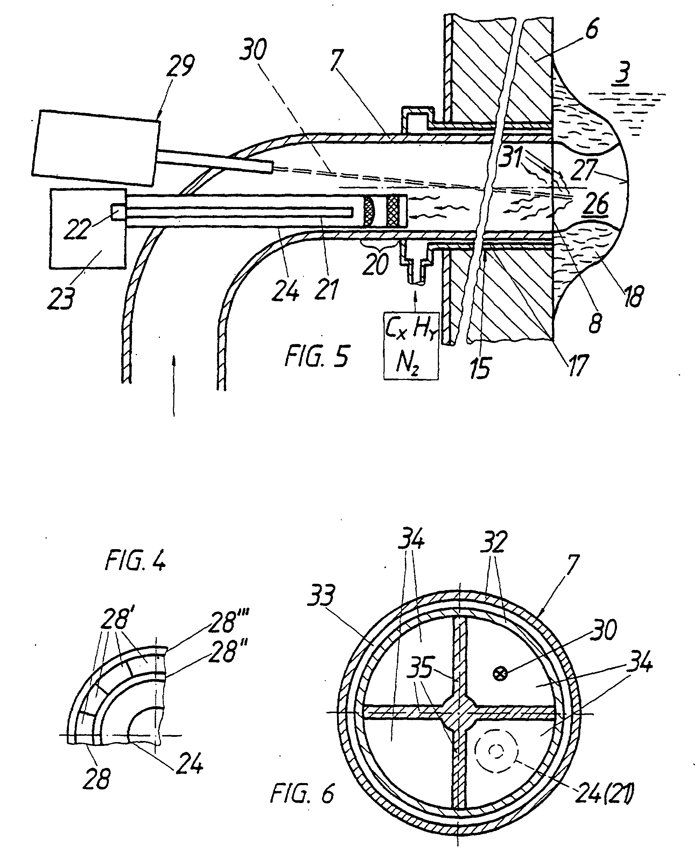

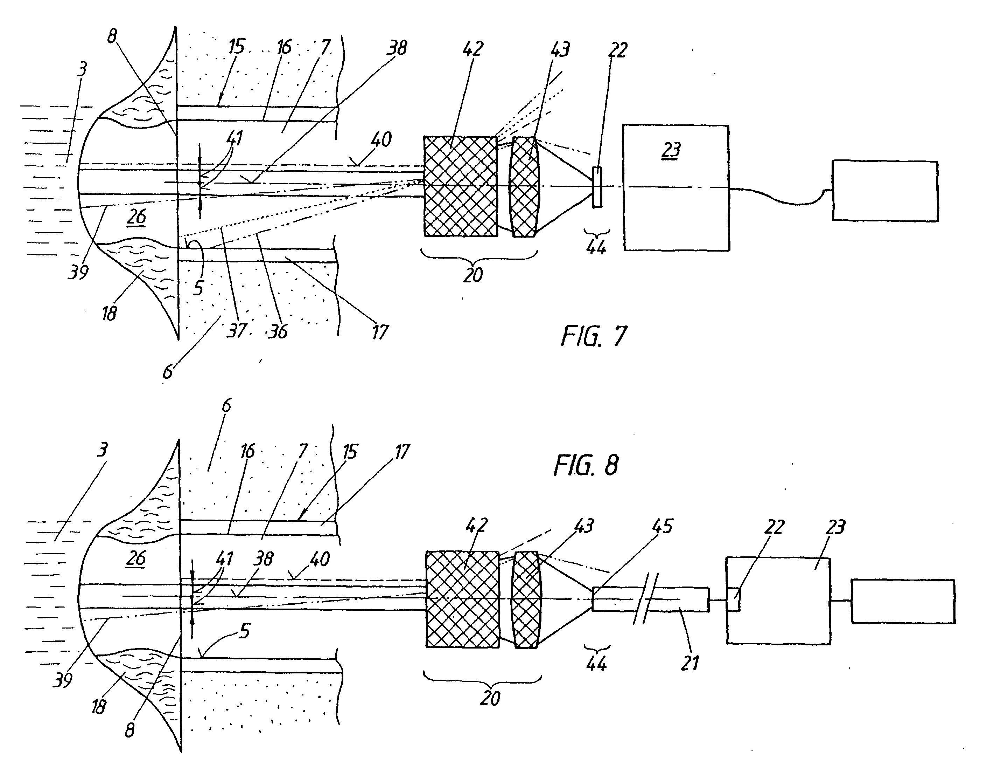

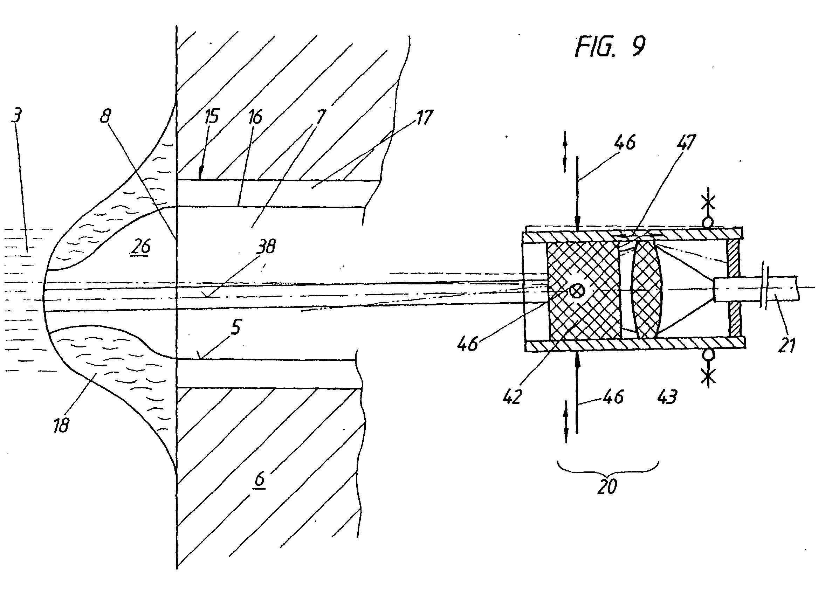

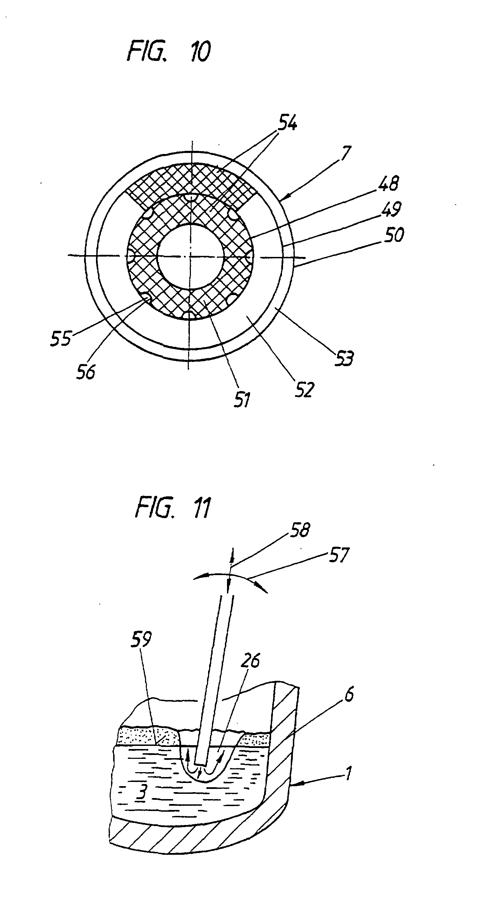

Die Erfindung ist nachfolgend anhand mehrerer in der Zeichnung schematisch dargestellter Ausführungsbeispiele näher erläutert, wobei Fig. 1 eine schematische Gesamtübersicht über eine erfindungsgemäße Einrichtung (teilweise geschnitten) und die Fig. 2 und 3 jeweils ein Detail der Fig. 1 in vergrößertem Maßstab in jeweils unterschiedlichen Ausgestaltungen veranschaulichen. In Fig. 4 ist in zu Fig. 3 analoger Darstellung eine spezielle Ausführungsform dargestellt. Die Fig. 5 und 6 veranschaulichen jeweils einen Schnitt quer zur Bildebene der Fig. 2 gemäß weiteren Ausführungsformen. Die Fig. 7 und 8 veranschaulichen in schematischer Weise die erfindungsgemäßen Strahlengänge. Fig. 9 zeigt eine bevorzugte Ausführungsform in zu Fig. 3 analoger Darstellung. Fig. 10 zeigt in zu Fig. 4 analoger Darstellung einen Querschnitt durch ein Gaszuführungsrohr. Fig. 11 betrifft eine weitere Ausführungsform.

Ein metallurgisches Gefäß 1, beispielsweise ein feuerfest ausgekleideter Konverter (es könnte auch ein Vakuumgefäß oder Elektroofen oder anderer Reaktor etc. sein) zur Aufnahme einer mit einer Schlackenschicht 2 bedeckten Stahlschmelze 3, weist in einer Höhe, die bei Normalfüllung des Konverters 1 unter dem Schmelzenbadspiegel 4 der Stahlschmelze 3 liegt, eine Öffnung 5 in einer Seitenwand 6 auf, in die eine Gaszuführungsleitung 7 eingesetzt ist, die mit einer Gasaustrittsöffnung 8 an der Innenseite 9 der Seitenwand 6 des Konverters 1 in dessen Innenraum 10 mündet. Über die Gaszuführungsleitung 7 können unterschiedliche Gase, beispielsweise Sauerstoff, Stickstoff, Luft, Erdgas oder Gemische davon, sowie gegebenenfalls auch Feststoffe, beispielsweise staubförmiger Kohlenstoff und/oder Schlackenbildner bzw. Stäube, eingebracht werden, wobei die genannten Gase gegebenenfalls für die Feststoffe als Trägergase fungieren. Die Gase werden in Tanks 11 gespeichert und bei Bedarf über Leitungen 12 entnommen. Die Feststoffe werden in einem oder in mehreren Fördergefäßen 13 gelagert bzw. vorhandenen Systemen entnommen und mittels eines Fördergases, beispielsweise Luft gemäß Fig. 1, dem Konverter 1 zugeleitet. Die Zusammensetzung der Gase bzw. Auswahl und Mengeneinstellung der Gase kann mit Hilfe eines schematisch dargestellten Ventilstandes 14 erfolgen.

Gemäß der in Fig. 2 dargestellten Ausführungsform ist das Ende der Gaszuführungsleitung 7 als Manteldüse 15 ausgebildet, wobei durch den ein Zentralrohr 16 der Manteldüse umgebenden Ringspalt 17 ein Kohlenwasserstoffgas, gegebenenfalls gemischt mit Stickstoff, in den Konverter 1 eingeleitet wird, wodurch es infolge Crackreaktionen zur Ausbildung eines ringförmigen und die Mündung der Manteldüse 15 schützenden Ansatzes 18 kommt. Das Ende der Gaszuführungsleitung 7 kann auch als einfaches Rohr (ohne Schutzgasmantel) ausgebildet sein, wenn die Haltbarkeit kein Kriterium darstellt.

In das Zentralrohr 16 mündet ein in Richtung der Achse der Manteldüse 15 und fluchtend zu dieser angeordnetes Zweigrohr 16', das mit einer Blende 19, die auch mehrere nebeneinander angeordnete Durchtrittsöffnungen für die elektromagnetischen Wellen aufweisen kann, ausgestattet ist. Hinter der Blende 19 ist ein als Sammellinse wirkendes optisches System 20 und hinter dem optischen System 20 ein Ende eines Lichtwellenleiters 21, beispielsweise eines Glasfaserleiters, vorgesehen. Der Lichtwellenleiter 21 führt zu einem auf elektromagnetische Wellen ansprechenden Detektor 22, der mit einem Verstärker und einer Auswerteelektronik 23 gekoppelt ist.

Der Lichtwellenleiter 21 und das optische System 20 sind vorteilhaft in einem Schutzrohr 24 eingebaut. In die Zweigleitung 16' läßt sich zweckmäßig inertes Gas einleiten, u.zw. über eine Leitung 25, wodurch eine Freihaltung des optischen Systems 20 von Staub gelingt.

Die Funktion der Einrichtung ist folgende:

Zur Durchführung einer Temperaturmessung wird durch die Gaszuführungsleitung 7 lediglich Gas - ohne Feststoffe -, vorzugsweise inertes Gas, in den Konverter 1 eingeblasen. Hierbei kommt es durch den Gasdruck zur Ausbildung eines von diesem Gas gefüllten Hohlraumes 26, der unmittelbar an den ringförmig ausgebildeten Ansatz 18 anschließt bzw. von diesem und der Schmelzenoberfläche 27 begrenzt ist. Die durch den Ansatz 18 sichergestellte freie Durchtrittsöffnung für das Gas soll ein Mindest-Ausmaß von etwa 0,2 bis 1,0 cm² ausmachen.

Von der Schmelzenoberfläche 27 der den gasgefüllten Hohlraum 26 begrenzenden Schmelze 3 werden elektromagnetische Wellen, insbesondere im Bereich des sichtbaren Lichts und im UV-Bereich, emittiert. Diese elektromagnetischen Wellen gelangen über die geöffnete Blende oder Klappe 19 und das optische System 20 zum Lichtwellenleiter 21 und über diesen zum Detektor 22. Eine Auswerteelektronik 23 gestattet die Feststellung der zu den auf natürliche Art emittierten elektromagnetischen Wellen äquivalenten Temperatur.

Gemäß der in Fig. 3 dargestellten Ausführungsform ragt das Schutzrohr 24 mit dem Lichtwellenleiter 21 direkt in die Gaszuführungsleitung 7, u.zw. im Bereich des als Manteldüse 15 ausgebildeten Endes derselben. Das Schutzrohr 24 kann mit Stickstoff gespült werden, was jedoch nicht im einzelnen dargestellt ist.

Gemäß Fig. 4, die einen Schnitt quer zur Längserstreckung einer Gaszuführungsleitung zeigt, ist die Gaszuführungsleitung 7 in ihrem Endbereich als Mehrkanaldüse ausgebildet. Im Zentrum der Mehrkanaldüse ist das Schutzrohr 24 und das optische System 20 mit dem Lichtwellenleiter 21 vorgesehen. Das Schutzrohr 24 ist peripher von zwei im radialen Abstand voneinander angeordneten Ringspalträumen 25, 26 umgeben, durch die beispielsweise Kohlenwasserstoffgase in den Konverter 1 eingeleitet werden können.

Der zwischen den beiden Ringspalträumen 28" und 28"' gebildete weitere Ringspaltraum 28 ist durch radiale Stege in mehrere Kanäle 28' unterteilt, die sich im Querschnitt gesehen über jeweils einen Teilumfangsbereich erstrecken. Durch diese Kanäle 28' können andere Gase, wie beispielsweise Sauerstoff, Inertgas oder Mischungen davon, in den Konverter eingeleitet werden.

Fig. 5 zeigt eine erfindungsgemäße Meßanordnung mit einer Laserstrahleneinrichtung 29, die zur Durchführung einer Schmelzenanalyse eingesetzt werden kann. Hierbei ist das Schutzrohr 24 mit dem Lichtwellenleiter 21 etwas außermittig der Gaszuführungsleitung 7 eingesetzt. Der von der Laserstrahleneinrichtung 29 erzeugte Laserstrahl 30 ist derart schräg in Richtung zur Gasaustrittsöffnung 8 gerichtet, daß er etwa das Zentrum der Gasaustrittsöffnung 8 durchstößt und im Konverterinneren Schmelze am Übergang Gasblase - Flüssigkeit verdampft. Die von der verdampften Schmelze emittierenden elektromagnetischen Wellen 31, die in Fig. 5 mit gewellten Pfeilen angedeutet sind, werden vom Lichtwellenleiter 21 erfaßt und über die Auswerteelektronik 23 ausgewertet. Vorzugsweise wird der Laserstrahl 30 durch eine Sammellinse fokussiert, wobei ein Brennfleck an der Öffnung 5 zwischen gasförmiger und flüssiger Fläche der Schmelze 3 gebildet wird. In Strahlrichtung ist die Einrichtung zweckmäßig beweglich ausgeführt, wodurch der Brennfleck optimal plaziert werden kann.

Die Gaszuführungsleitung 7 ist in ihrem Endbereich als Mantelringdüse ausgebildet, wobei durch den Ringraum bzw. Ringspalt 17 Kohlenwasserstoffgase, inerte Gase oder Mischungen davon in den Konverter 1 eingeleitet werden.

Fig. 6 veranschaulicht einen Querschnitt durch den Endbereich einer Gaszuführungsleitung 7 gemäß einer leicht modifizierten Form. Außenseitig ist die Gaszuführungsleitung 7 von einem Doppelmantel 32 gebildet, wobei durch den vom Doppelmantel gebildeten Ringraum 33 Kohlenwasserstoffgase, Stickstoff etc. eingeleitet wird. Der Innenraum der Gaszuführungsleitung 7 ist durch sich in Längserstreckung und radial gerichtete Wände 35 mehrfach geteilt, u.zw. gemäß dem dargestellten Ausführungsbeispiel in vier etwa gleich große Räume 34 geteilt. Durch einen der Räume 34 ist der Laserstrahl 30 in das Innere des Konverters 1 gerichtet und durch einen zweiten Raum 34 ragt das Schutzrohr 24 mit dem Linsensystem mit dem Lichtwellenleiter 21. Jeder der Räume 34 kann mit unterschiedlichen Gasen beaufschlagt werden, beispielsweise mit Sauerstoff oder Inertgas oder Mischungen davon.

Aus den Fig. 7 und 8 sind die erfindungsgemäß bevorzugten Strahlengänge, die schematisch dargestellt sind, zu ersehen. Vom Randbereich 35 des Hohlraumes 26 bzw. der Öffnung 5 ausgehende elektromagnetische Wellen 36 und insbesondere von dem Ansatz 18 reflektierte elektromagnetische Wellen 37 sowie schräg zur optischen Achse 38 des optischen Systems 20 verlaufende elektromagnetische Wellen 39 und elektromagnetische Wellen 40, die außerhalb eines von der optischen Achse 38 des optischen Systems 20 gezogenen Grenzradius 41 vorhanden sind, werden von einer Detektierung ausgeschlossen, indem diese elektromagnetischen Wellen von einer Wellenstreueinrichtung 42, die beispielsweise als Zerstreuungs- und Sammellinsensystem ausgebildet ist, von der optischen Achse 38 des optischen Systems 20 weggebrochen werden.

Der Wellenstreueinrichtung 42 ist eine Wellenbündeleinrichtung 43 nachgeordnet, mit der die etwa parallel zur optischen Achse 38 des optischen Systems 20 gerichteten elektromagnetischen Wellen fokussiert werden. Die schräg zur optischen Achse 38 des optischen Systems 20 gerichteten und außerhalb eines von der optischen Achse 38 des optischen Systems 20 gezogenen Grenzradius 41 vorhandenen elektromagnetischen Wellen 39, 40 sind jedoch von dieser Fokussierung nicht mitumfaßt.

Der Unterschied zwischen der in Fig. 7 dargestellten Variante von der in Fig. 8 dargestellten Variante ist darin zu sehen, daß einmal der Detektor 22 direkt im Fokussierungsbereich 44 der Wellenbündeleinrichtung 43 liegt (Fig. 7) und einmal gemäß Fig. 8 im Fokussierungsbereich ein Einlaß 45 eines Lichtwellenleiters liegt, der zu einem Detektor mit Auswerteelektronik führt.

Gemäß der in Fig. 9 dargestellten Ausführungsform ist das optische System 20 - bestehend aus Wellenstreueinrichtung 42 und Wellenbündeleinrichtung 43 - im Zentralrohr 16 schwenkbar gelagert, u.zw. vorzugsweise derart schwenkbar gelagert, daß mit der optischen Achse 38 des optischen Systems 20 jeder Punkt im Querschnitt der Öffnung 5 erreicht werden kann. Eine solche bewegliche Lagerung kann mittels mehrerer am optischen System angreifender Druckmittelzylinder 46, die in Fig. 9 mit Pfeilen angedeutet sind, oder mit einer kardanischen Lagerung verwirklicht werden. Hierdurch gelingt es, die optische Achse 38 des optischen Systems 20 derart auszurichten, daß sie selbst bei einseitigem Ansatzwachstum, das in Fig. 9 veranschaulicht ist, gegen die Schmelze 3 gerichtet werden kann, wodurch durch den Ansatz 18 verursachte Meßwertverfälschungen vermieden werden können. In diesem Fall wird das optische System 20 so lange verschwenkt, bis die Intensität der emittierenden elektromagnetischen Wellen bei der Auswertung derselben ein Maximum ergibt. Dies ist ein Kriterium dafür, daß die optische Achse des optischen Systems 20 tatsächlich gegen die Schmelze 3 und nicht etwa gegen den Randbereich des Ansatzes 18 oder den Randbereich der Öffnung 5 gerichtet ist. Die Bewegung des optischen Systems 20 kann mit Hilfe eines elektromechanischen Antriebs erfolgen, der das optische System 20 automatisch so einstellt, daß ein Intensitätsmaximum ausgebildet wird. Weiters kann auch eine axiale Verschiebbarkeit des optischen Systems 20 vorgesehen sein, wie der Doppelpfeil 47 veranschaulicht, wofür ebenfalls Elektromotoren oder Druckmittelzylinder vorgesehen sein können.

In Fig. 10 ist in zu Fig. 4 analoger Darstellung ein Querschnitt durch ein Gaszuführungsrohr gezeigt, das von vier konzentrisch angeordneten Zylinderrohren 24, 48, 49, 50 gebildet ist, wobei zwischen den Zylinderrohren jeweils Zwischenräume 51, 52, 53 vorhanden sind. Das innerste Rohr 24 dient als Gaszuführungsrohr für die Durchführung der Messung. Hier befindet sich das optische System 20 und der Wellenleiter 21 sowie gegebenenfalls der Detektor 22. Der radial daran anschließende Zwischenraum 51 zwischen den Zylinderrohren 24 und 48 ist mit Feuerfestmaterial 54 gefüllt, wobei jedoch Nuten 55 am Außenumfang des Feuerfestmaterials vorgesehen sind, die gegebenenfalls von Blechmänteln 56 ausgekleidet sind. Über diese Nuten wird Schutzgas, z.B. CH₄, CH₄ + N₂ etc., zum Ende der Gaszuführungsleitung 7 geführt. Der daran radial anschließende Ringraum 52 ist etwa zu einem Viertel in Umfangsrichtung mit Feuerfestmaterial 54 gefüllt, die restlichen drei Viertel des Ringraumes 52 sind frei und dienen zur Zufuhr von Sauerstoff oder Sauerstoff gemischt mit anderen Gasen. Der radial äußerste Ringraum 53 dient wiederum zur Zuführung eines Schutzgases.

Gemäß der in Fig. 11 dargestellten Ausführungsform wird ein Gaszuführungsrohr 7, in dem das optische System 20 und die Signalaufnehmer (Lichtwellenleiter 21 und/oder Detektor 22) eingebaut sind, durch einen nicht näher dargestellten Bewegungsmechanismus, der Bewegungen in Richtungen der in der Fig. 11 dargestellten Pfeile 57, 58 gestattet, von oben in die Schmelze 3 durch die Oberfläche 59 derselben bewegt, wobei es in der Schmelze 3 zur Ausbildung eines gasgefüllten Hohlraumes 26 kommt. Auch hier kann das Ende des Gaszuführungsrohres 7 als Manteldüse zur Bildung eines Schutzgasmantels ausgebildet sein.

Die Messung kann nach zwei verschiedenen Grundprinzipien durchgeführt werden, u.zw. einmal mit Hilfe eines Pyrometers und einmal mit Hilfe eines Spektrometers. Die Auswertung erfolgt anschließend über spezielle, für beide Systeme unterschiedliche Auswerteelektroniken.

Die emittierende Strahlung unterscheidet sich bei reiner Temperaturmessung von der für eine Analyse der Schmelze. Bei einer Schmelzenanalyse wird das durch einen Laser erzeugte, von einem Plasma emittierte Spektrum betrachtet (UV-Bereich).

## Patentansprüche

1. Verfahren zur Bestimmung von aus dem Inneren einer Schmelze (3), insbesondere einer Metallschmelze, ausgehenden elektromagnetischen Wellen, insbesondere im Bereich des sichtbaren Lichts und des angrenzenden UV-Bereiches und Infrarotbereiches, wobei in der Schmelze (3) durch Einblasen von Gas ein gasgefüllter Hohlraum (26) gebildet wird und von der Schmelze (3) emittierende elektromagnetische Wellen (31) durch das eingeblasene Gas hindurch beobachtet und ausgewertet werden, indem die elektromagnetischen Wellen über ein optisches System (20) einem Detektor (22) zur Bestimmung der Temperatur und/oder der chemischen Zusammensetzung zugeleitet werden, **dadurch gekennzeichnet, daß** schräg zur optischen Achse (38) des optischen Systems (20) gerichtete und vom Randbereich des Hohlraumes (26) ausgehende elektromagnetische Wellen (39, 36, 37) von einer Detektierung ausgeschlossen werden, indem die emittierenden elektromagnetischen Wellen (31) von schräg zur optischen Achse (38) des optischen Systems (20) gerichteten und außerhalb eines von der optischen Achse (38) des optischen Systems (20) gezogenen Grenzradius (41) vorhandenen elektromagnetischen Wellen (36, 37, 39, 40) befreit werden, indem diese elektromagnetischen Wellen (36, 37, 39, 40) in einer Wellenstreueinrichtung (42) des optischen Systems (20), vorzugsweise einem Zerstreuungs- und Sammellinsensystem, von der optischen Achse (38) des optischen Systems (20) weggebrochen werden und nur etwa parallel zur optischen Achse (38) des optischen Systems (20) gerichtete elektromagnetische Wellen zu einem dem optischen System (20) nachgeordneten Detektor (22) gelangen.

2. Verfahren nach Anspruch 1, **dadurch gekennzeichnet, daß** das optische System (20) unter Ausrichtung seiner optischen Achse (38) gegenüber dem Hohlraum (26) bewegt wird, u.zw. so lange bewegt wird, bis die Intensität der emittierenden elektromagnetischen Wellen bei der Auswertung derselben ein Maximum ergibt.

3. Verfahren nach Anspruch 1 oder 2, **dadurch gekennzeichnet, daß** der Wellenstreueinrichtung (42) eine Wellenbündeleinrichtung (43), wie eine Sammellinse oder ein Sammellinsensystem, nachgeordnet ist und die etwa parallel zur optischen Achse (38) des optischen Systems (20) gerichteten elektromagnetischen Wellen von der Wellenbündeleinrichtung (43) fokussiert und dem Detektor (22) direkt oder über einen Lichtwellenleiter (21) zugeleitet werden, die schrägen und außerhalb eines Grenzradius vorhandenen Wellen (36, 37, 39, 40) jedoch von dieser Fokussierung nicht mitumfaßt werden.

4. Verfahren nach Anspruch 1 oder 2, **dadurch gekennzeichnet, daß** sowohl die Wellenstreueinrichtung (42) als auch die nachgeordnete Wellenbündeleinrichtung (43) unter Ausrichtung ihrer optischen Achse (38) gegenüber dem Hohlraum (26) bewegt werden, bis die Intensität der emittierenden elektromagnetischen Wellen bei der Auswertung derselben ein Maximum ergibt.

5. Verfahren nach Anspruch 4, **dadurch gekennzeichnet, daß** über den gasgefüllten Hohlraum der Schmelze (3) Energie zugeführt und durch die zugeführte Energie ein Teil der Schmelze verdampft wird, insbesondere dadurch, daß das eingeblasene Gas mit der Schmelze (3) eine chemische Reaktion eingeht, die zum Verdampfen eines Teiles der Schmelze (3) führt.

6. Verfahren nach einem oder mehreren der Ansprüche 1 bis 5, **dadurch gekennzeichnet, daß** das zur Bildung des gasgefüllten Hohlraumes (26) eingeblasene Gas an der Eintrittsstelle in die Schmelze (3) von einem Gasmantel oder mehreren Gasmänteln umgeben ist, der bzw. die ein kohlenwasserstoffhältiges Schutzmedium, vorzugsweise gemischt mit Inertgas, enthält bzw. enthalten.

7. Verfahren nach einem oder mehreren der Ansprüche 1 bis 6, **dadurch gekennzeichnet, daß** die Bestimmung der Temperatur bzw. chemischen Analyse der Schmelze (3) kombiniert wird mit vorgerechneten bzw. gemessenen Parametern, wie einer Kohlenstoffbilanz der Abgasanalyse oder einer groben Berechnung der Analyse der Schmelze zum Zeitpunkt der Messung.

8. Verfahren nach einem oder mehreren der Ansprüche 1 bis 6, **dadurch gekennzeichnet, daß** nur der Gehalt einzelner Elemente der Schmelze (3), wie z.B. bei Eisenschmelzen der Mn-, Cr-, C-Gehalt etc., ermittelt werden und daraus der Gehalt der anderen in der Schmelze (3) und auch Schlackenschmelze (2) vorhandenen Elemente bzw. Verbindungen berechnet wird.

9. Verfahren nach einem oder mehreren der Ansprüche 1 bis 8, **dadurch gekennzeichnet, daß** während der Messung im Hohlraum (26) bzw. knapp davor durch Einleiten eines Gasgemisches eine Temperatur eingestellt wird, die möglichst nahe an der Ist-Temperatur der Schmelze (3) liegt.

10. Verfahren nach einem oder mehreren der Ansprüche 1 bis 9, **dadurch gekennzeichnet, daß** mit einem Gas oder mehreren verschiedenen Gasen, die in die Schmelze (3) eingeleitet werden, die chemische Analyse der Schmelze gezielt verändert und die Schmelze (3) bzw. Schmelze (3) und Schlacke (2) durchmischt werden.

11. Verfahren nach einem oder mehreren der Ansprüche 1 bis 10, **dadurch gekennzeichnet, daß** der gasgefüllte Hohlraum (26) an der Schmelzenoberfläche (59) gebildet wird.

12. Einrichtung zur Durchführung des Verfahrens nach einem oder mehreren der Ansprüche 1 bis 11, mit
• einem eine Schmelze (3) aufnehmenden Gefäß (1),
• einer zu einer Öffnung (5) des Gefäßes (1) führenden Gaszuführungsleitung (7) mit einer gegen die Öffnung (5) und damit gegen die Schmelze (3) gerichteten Gasaustrittsöffnung (8),
• einem optischen System (20) zur Beobachtung der Gasaustrittsöffnung (8),
• einem Detektor (22) zur Registrierung von aus der Schmelze (3) emittierenden elektromagnetischen Wellen (31),
• gegebenenfalls einem die elektromagnetischen Wellen (31) zum Detektor (22) führenden Wellenleiter (7, 21),
**gekennzeichnet durch**
• eine optische Wellenstreueinrichtung (42), vorzugsweise ein Zerstreuungs-Sammellinsensystem.

13. Einrichtung nach Anspruch 12, **gekennzeichnet durch** eine gegenüber dem metallurgischen Gefäß (1) unter Ausrichtung der optischen Achse (38) bewegbare, vorzugsweise schwenkbare, Anordnung des optischen Systems (20).

14. Einrichtung nach Anspruch 12 oder 13, **gekennzeichnet durch**
• eine der Wellenstreueinrichtung (42) nachgeordnete Wellenbündeleinrichtung (43), wie eine Sammellinse oder ein nachgeordnetes Sammellinsensystem, und
• einen im Fokussierungsbereich (44) der Wellenbündeleinrichtung liegenden Detektor (22) oder einen dort angeordneten und zu einem Detektor (22) führenden Lichtwellenleiter (21).

15. Einrichtung nach einem oder mehreren der Ansprüche 12 oder 14, **dadurch gekennzeichnet, daß** ein Schutzrohr (24) für das optische System mit einer Gasspülung (25), insbesondere einer das Linsensystem (20) an der Frontseite reinigenden Gasspülung, vorgesehen ist.

16. Einrichtung nach einem oder mehreren der Ansprüche 12 bis 15, **dadurch gekennzeichnet, daß** die Wellenstreueinrichtung (42) gegenüber der gegen die Schmelze (3) gerichteten Gasaustrittsöffnung (8) schwenkbar ist, wobei der Schnittpunkt der optischen Achse (38) der Wellenstreueinrichtung (42) mit der Querschnittsfläche der Gasaustrittsöffnung (8) innerhalb deren Querschnittsfläche verstellbar ist.

17. Einrichtung nach Anspruch 16, **dadurch gekennzeichnet, daß** sowohl die Wellenstreueinrichtung (42) als auch die Wellenbündeleinrichtung (43) schwenkbar gelagert sind.

18. Einrichtung nach Anspruch 16 oder 17, **dadurch gekennzeichnet, daß** die schwenkbare Lagerung als kardanische Lagerung ausgebildet ist.

19. Einrichtung nach einem oder mehreren der Ansprüche 12 bis 18, **dadurch gekennzeichnet, daß** im Fokussierungsbereich (44) der Wellenbündeleinrichtung (43) ein Einlaß (45) eines Lichtwellenleiters (21) angeordnet ist.

20. Einrichtung nach einem oder mehreren der Ansprüche 12 bis 19, **dadurch gekennzeichnet, daß** im Fokussierungsbereich (44) der Wellenbündeleinrichtung (43) der Detektor (22) angeordnet ist.

21. Einrichtung nach einem oder mehreren der Ansprüche 12 bis 20, **dadurch gekennzeichnet, daß** das Ende der Gaszuführungsleitung (7) als Zwei- oder Mehrrohrdüse (15) ausgebildet ist, deren Mantelringraum (17) bzw. -räume an eine ein Kohlenwasserstoffgas zuführende Leitung anschließbar ist (sind).

22. Einrichtung nach einem oder mehreren der Ansprüche 12 bis 21, **dadurch gekennzeichnet, daß** das Ende der Gaszuführungsleitung (7) von einer Mehrkanaldüse gebildet ist, deren Düsenöffnungen an eine oder mehrere Zuführungsleitungen für Kohlenwasserstoff, Kohlenmonoxid, Kohlendioxid, Inertgas, Dampf, Öl oder Wasser bzw. Mischungen davon anschließbar sind.

23. Einrichtung nach einem oder mehreren der Ansprüche 12 bis 22, **dadurch gekennzeichnet, daß** eine gegen die Gasaustrittsöffnung (8) der Gaszuführungsleitung (7) gerichtete Laserstrahleinrichtung (29) vorgesehen ist.

24. Einrichtung nach Anspruch 23, **dadurch gekennzeichnet, daß** der Laserstrahleinrichtung (29) eine Fokussiereinrichtung zugeordnet ist.

25. Einrichtung nach einem oder mehreren der Ansprüche 12 bis 24, **gekennzeichnet durch** eine in die Schmelze (3) eintauchende Gaszuführungsleitung (7) mit Wellenstreueinrichtung (42).

26. Verfahren zum Betrieb einer Einrichtung nach einem oder mehreren der Ansprüche 12 bis 25, **dadurch gekennzeichnet, daß** zum Schutz des in das Gefäß (1) mündenden Teiles der Einrichtung (7,21,23, 15) die Zufuhr eines Schutzmediums geregelt wird, indem mit zunehmendem Angriff der Schmelze (3), d.h. mit steigender Temperatur bzw. Überhitzung der Schmelze (3), die Zufuhr von kohlenwasserstoffhältigem Schutzmedium stufenweise oder kontinuierelich verstärkt wird.

## Claims

1. A process for determining electromagnetic waves emanating from the interior of a melt (3), in particular of a molten metal, in particular in the range of visible light and of the neighbouring UV and infrared ranges, wherein a gas-filled cavity (26) is formed in the melt (3) by blowing in gas, and electromagnetic waves (31) emitted from the melt (3) are observed through the gas that has been blown in and are evaluated by the electromagnetic waves being fed via an optical system (20) to a detector (22) for determining the temperature and/or the chemical composition, **characterised in that** electromagnetic waves (39, 36, 37) directed obliquely relative to the optical axis (38) of the optical system (20) and emanating from the marginal region of the cavity (26) are excluded by a detection stage by the electromagnetic waves (31) emitted being freed of electromagnetic waves (36, 37, 39, 40) directed obliquely relative to the optical axis (38) of the optical system (20) and present outside a limiting radius (41) drawn from the optical axis (38) of the optical system (20), by these electromagnetic waves (36, 37, 39, 40) being refracted away from the optical axis (38) of the optical system (20) in a wave-scattering device (42) pertaining to the optical system (20), preferably in a diverging-lens and converging-lens system, and by only electromagnetic waves directed approximately parallel to the optical axis (38) of the optical system (20) arriving at a detector (22) arranged downstream of the optical system (20).

2. Process according to Claim 1, **characterised in that** the optical system (20) is moved subject to alignment of its optical axis (38) in relation to the cavity (26) and specifically is moved until such time as the intensity of the electromagnetic waves emitted shows a maximum when said electromagnetic waves are evaluated.

3. Process according to Claim 1 or 2, **characterised in that** a wave-bundling device (43) such as a converging lens or a converging-lens system is arranged downstream of the wave-scattering system (42), and the electromagnetic waves directed approximately parallel to the optical axis (38) of the optical system (20) are focused by the wave-bundling device (43) and are fed to the detector (22) directly or via an optical waveguide (21) but the oblique waves (36, 37, 39, 40) that are present outside a limiting radius are not encompassed by this focusing.

4. Process according to Claim 1 or 2, **characterised in that** both the wave-scattering device (42) and the wave-bundling device (43) arranged downstream are moved subject to alignment of their optical axis (38) in relation to the cavity (26) until the intensity of the electromagnetic waves emitted shows a maximum when said electromagnetic waves are evaluated.

5. Process according to Claim 4, **characterised in that** energy is supplied via the gas-filled cavity of the melt (3) and some of the melt is vaporized by the supplied energy, in particular **in that** the gas that has been blown in enters into a chemical reaction with the melt (3), which results in vaporization of some of the melt (3).

6. Process according to one or more of Claims 1 to 5, **characterised in that** the gas that is blown in for the purpose of forming the gas-filled cavity (26) is surrounded at the point of entry into the melt (3) by a gas jacket or by several gas jackets which contains/ contain a hydrocarbon-containing protective medium, preferably mixed with inert gas.

7. Process according to one or more of Claims 1 to 6, **characterised in that** the determination of the temperature or of the chemical analysis of the melt (3) is combined with calculated or measured parameters such as a carbon balance of the waste-gas analysis or a rough calculation of the analysis of the melt at the time of measurement.

8. Process according to one or more of Claims 1 to 6, **characterised in that** only the content of individual elements of the melt (3) such as, in the case of iron melts for example, the content of Mn, Cr, C etc are ascertained and the content of the other elements or compounds that are present in the melt (3) and also in the molten slag (2) is calculated therefrom.

9. Process according to one or more of Claims 1 to 8, **characterised in that** during the measurement in the cavity (26) or just before this measurement a temperature that lies as close as possible to the actual temperature of the melt (3) is adjusted by introducing a gas mixture.

10. Process according to one or more of Claims 1 to 9, **characterised in that** the chemical analysis of the melt is changed purposefully with a gas or with several different gases which are introduced into the melt (3) and the melt (3) or melt (3) and slag (2) are mixed thoroughly.

11. Process according to one or more of Claims 1 to 10, **characterised in that** the gas-filled cavity (26) is formed on the surface (59) of the melt.

12. A device for implementing the process according to one or more of Claims 1 to 11, with
· a vessel (1) receiving a melt (3),
· a gas supply line (7) leading to an opening (5) in the vessel (1), with a gas outlet (8) directed towards the opening (5) and hence towards the melt (3),
· an optical system (20) for observing the gas outlet (8),
· a detector (22) for registering electromagnetic waves (31) emitted from the melt (3),
· optionally, a waveguide (7, 21) guiding the electromagnetic waves (31) to the detector (22),
**characterised by**
· an optical wave-scattering device (42), preferably a diverging/converging lens system.

13. Device according to Claim 12, **characterised by** an arrangement of the optical system (20) that is capable of being moved, preferably capable of being swivelled, in relation to the metallurgical vessel (1) subject to alignment of the optical axis (38).

14. Device according to Claim 12 or 13, **characterised by**
· a wave-bundling device (43) arranged downstream of the wave-scattering device (42); such as a converging lens or a converging-lens system arranged downstream, and
· a detector (22) situated in the focusing region (44) of the wave-bundling device or an optical waveguide (21) arranged there and leading to a detector (22).

15. Device according to one or more of Claims 12 or 14, **characterised in that** a protective tube (24) for the optical system is provided with a gas flushing (25), in particular with a gas flushing cleaning the lens system (20) on the front side.

16. Device according to one or more of Claims 12 to 15, **characterised in that** the wave-scattering device (42) is capable of being swivelled in relation to the gas outlet (8) directed towards the melt (3), the point of intersection of the optical axis (38) of the wave-scattering device (42) being adjustable by the cross-sectional area of the gas outlet (8) within the cross-sectional area thereof.

17. Device according to Claim 16, **characterised in that** both the wave-scattering device (42) and the wave-bundling device (43) are supported so as to be capable of swivelling.

18. Device according to Claim 16 or 17, **characterised in that** the swivelling support takes the form of a cardanic support.

19. Device according to one or more of Claims 12 to 18, **characterised in that** an inlet (45) of an optical waveguide (21) is arranged in the focusing region (44) of the wave-bundling device (43).

20. Device according to one or more of Claims 12 to 19, **characterised in that** the detector (22) is arranged in the focusing region (44) of the wave-bundling device (43).

21. Device according to one or more of Claims 12 to 20, **characterised in that** the end of the gas supply line (7) takes the form of a two-pipe or multi-pipe tuyere (15), the encased annular space (17) or spaces of which is/are capable of being connected to a line supplying a hydrocarbon gas.

22. Device according to one or more of Claims 12 to 21, **characterised in that** the end of the gas supply line (7) is constituted by a multi-channel tuyere, the tuyere openings of which are capable of being connected to one or more supply lines for hydrocarbon, carbon monoxide, carbon dioxide, inert gas, steam, oil or water or mixtures thereof.

23. Device according to one or more of Claims 12 to 22, **characterised in that** a laser-beam device (29) directed towards the gas outlet (8) of the gas supply line (7) is provided.

24. Device according to Claim 23, **characterised in that** a focusing device is assigned to the laser-beam device (29).

25. Device according to one or more of Claims 12 to 24, **characterised by** a gas supply line (7) immersed in the melt (3) with wave-scattering device (42).

26. A process for operating a device according to one or more of Claims 12 to 25, **characterised in that** with a view to protecting the part of the device (7, 21, 23, 15) leading into the vessel (1) the supply of a protective medium is regulated by the supply of hydrocarbon-containing protective medium being intensified in stepwise manner or continuously with increasing corrosion of the melt (3), i.e. with rising temperature or overheating of the melt (3).

## Revendications

1. Procédé pour déterminer les ondes électromagnétiques émanant de l'intérieur d'une masse en fusion (3), au particulier de métal en fusion, en particulier dans la plage de la lumière visible et les plages adjacentes dans le domaine des ultraviolets et de l'infrarouge, dans lequel on forme dans la masse en fusion (3) une cavité (26) remplie de gaz par soufflage d'un gaz, et l'on observe et l'on évalue les ondes électromagnétiques émises par la masse en fusion (3) à travers le gaz introduit, en amenant les ondes électromagnétiques via un système optique (20) à un détecteur (22) pour déterminer la température et/ou la composition chimique, **caractérisé en ce que** les ondes électromagnétiques (39, 36, 37) dirigées en oblique par rapport à l'axe optique (38) du système optique (20) et émanant de la région de bordure de la cavité (26) sont exclues d'une détection, en supprimant hors des ondes électromagnétiques émises (31) les ondes dirigées en oblique par rapport à l'axe optique (38) du système optique (20) et présentes à l'extérieur d'un rayon limite (41) tiré depuis l'axe optique (38) du système optique (20), en diffractant ces ondes électromagnétiques (36, 37, 39, 40) en éloignement de l'axe optique (38) du système optique (20), dans un dispositif de diffusion d'ondes (42) du système optique (20), de préférence un système à lentilles divergentes et convergentes, et seules des ondes électromagnétiques dirigées approximativement parallèlement à l'axe optique (38) du système optique (20) parviennent à un détecteur (22) agencé en aval du système optique (20).

2. Procédé selon la revendication 1, **caractérisé en ce que** le système optique (20) est déplacé, en alignant son axe optique (38) par rapport à la cavité (26), et cela aussi longtemps que l'intensité des ondes électromagnétiques émises produisent un maximum lors de leur évaluation.

3. Procédé selon l'une ou l'autre des revendications 1 et 2, **caractérisé en ce que** le dispositif de diffusion d'ondes (42) est suivi d'un dispositif de focalisation d'ondes (43), comme une lentille convergente ou un système de lentilles convergentes, qui focalise les ondes électromagnétiques dirigées approximativement parallèlement à l'axe optique (38) du système optique (20) provenant du système de focalisation d'ondes (43), et les amène au détecteur (22), soit directement soit via un guide d'ondes (21), cependant que les ondes obliques (36, 37, 39, 40) présentes à l'extérieur d'un rayon limite ne sont pas concernées par cette focalisation.

4. Procédé selon l'une ou l'autre des revendications 1 et 2, **caractérisé en ce que** le dispositif de diffusion d'ondes (42) tout comme le dispositif de focalisation d'ondes (43) qui le suit sont déplacés en alignant leurs axes optiques (38) par rapport à la cavité (26) jusqu'à ce que l'intensité des ondes électromagnétiques émises produise un maximum lors de leur évaluation.

5. Procédé selon la revendication 4, **caractérisé en ce que** l'on apporte de l'énergie à la masse en fusion (3) via la cavité remplie de gaz, et l'on vaporise avec l'énergie apportée une partie de la masse en fusion, en particulier du fait que le gaz injecté entre en réaction chimique avec la masse en fusion (3), réaction qui mène à vaporiser une partie de la masse en fusion (3).

6. Procédé selon l'une ou plusieurs des revendications 1 à 5, **caractérisé en ce que** le gaz injecté pour former la cavité remplie de gaz (26) est entouré, au niveau de l'emplacement de pénétration dans la masse en fusion (3), par une enveloppe gazeuse, ou plusieurs enveloppes gazeuses qui contient/contiennent un milieu protecteur contenant des hydrocarbures, de préférence en mélange avec un gaz inerte.

7. Procédé selon l'une ou plusieurs des revendications 1 à 6, **caractérisé en ce que** la détermination de la température, ou l'analyse chimique de la masse en fusion (3) est combinée avec des paramètres préalablement calculés ou mesurés, comme un bilan carbone de l'analyse des gaz sortants, ou avec un calcul grossier de l'analyse de la masse en fusion à l'instant de la mesure.

8. Procédé selon l'une ou plusieurs des revendications 1 à 6, **caractérisé en ce que** l'on détermine uniquement la teneur des éléments individuels dans la masse en fusion (3), comme par exemple la teneur en Mn-, Cr-, C- dans le cas de fonte de fer, et **en ce que** l'on calcule à partir de ces données la teneur des autres éléments ou composés présents dans la masse en fusion (3) ainsi que dans le laitier en fusion (2).

9. Procédé selon l'une ou plusieurs des revendications 1 à 8, **caractérisé en ce que** pendant la mesure, on établit dans la cavité (26) ou à brève distance devant celle-ci, par apport d'un mélange gazeux une température aussi proche que possible de la température réelle de la masse en fusion (3).

10. Procédé selon l'une ou plusieurs des revendications 1 à 9, **caractérisé en ce qu'**au moyen d'un gaz ou de plusieurs gaz différents que l'on introduit dans la masse en fusion (3), on modifie de façon ciblée l'analyse chimique de la masse en fusion, et on mélange la masse en fusion (3), ou la masse en fusion (3) et le laitier (2).

11. Procédé selon l'une ou plusieurs des revendications 1 à 10, **caractérisé en ce que** la cavité remplie de gaz (26) est formée au niveau de la surface de la masse en fusion (59).

12. Appareil pour mettre en oeuvre le procédé selon l'une ou plusieurs des revendications 1 à 11, comprenant :
- un récipient (1) qui reçoit la masse en fusion (3),
- une conduite d'amenée de gaz (7) qui mène à une ouverture (5) du récipient (1), avec une ouverture de sortie de gaz (8) dirigée vers l'ouverture (5) et ainsi vers la masse en fusion (3),
- un système optique (20) pour observer l'ouverture de sortie de gaz (8),
- un détecteur (22) pour enregistrer les ondes électromagnétiques (31) émises hors de la masse en fusion (3),
- le cas échéant un guide d'ondes (7, 21) menant les ondes électromagnétiques (31) au détecteur (22),
**caractérisé par**
- un dispositif de diffusion d'ondes (42), de préférence un système à lentilles divergentes/convergentes.

13. Appareil selon la revendication 12, **caractérisé en ce que** le système optique (20) est agencé de façon mobile, et de préférence pivotant, par rapport au récipient métallurgique (7) avec orientation de l'axe optique (38).

14. Appareil selon l'une ou l'autre des revendications 12 et 13, **caractérisé par** :
- un dispositif de focalisation d'ondes (43) agencé en aval du dispositif de diffusion d'ondes (42), comme une lentille convergente, ou un système de lentilles convergentes placé en aval, et
- un détecteur (22) disposé dans la zone de focalisation (44) du dispositif de focalisation d'ondes, ou un guide d'ondes (21) agencé à cet emplacement et menant à un détecteur (22).

15. Appareil selon l'une ou plusieurs des revendications 12 à 14, **caractérisé en ce qu'**il est prévu un tube protecteur (24) pour le système optique, avec un rinçage de gaz (25), en particulier un rinçage de gaz qui nettoie le système de lentille (20) au niveau de la face frontale.

16. Appareil selon l'une ou plusieurs des revendications 12 à 15, **caractérisé en ce que** le dispositif de diffusion d'ondes (42) est capable de pivoter par rapport à l'ouverture de sortie de gaz (8) dirigée vers la masse en fusion (3), et **en ce que** le point d'intersection de l'axe optique (38) du dispositif de diffusion d'ondes (42) avec la surface transversale de l'ouverture de sortie de gaz (8) est réglable l'intérieur de la surface transversale.

17. Appareil selon la revendication 16, **caractérisé en ce que** le dispositif de diffusion d'ondes (42) tout comme le dispositif de focalisation d'ondes (43) sont montés avec faculté de pivotement.

18. Appareil selon l'une ou l'autre des revendications 16 et 17, **caractérisé en ce que** le montage pivotant est réalisé sous la forme d'un montage à la cardan.

19. Appareil selon l'une ou plusieurs des revendications 12 à 18, **caractérisé en ce qu'**une entrée (45) d'un guide d'ondes (21) est agencée dans la zone de focalisation (44) du dispositif de focalisation d'ondes (43).

20. Appareil selon l'une ou plusieurs des revendications 12 à 19, **caractérisé en ce que** le détecteur (22) est agencé dans la zone de focalisation (44) du dispositif de focalisation d'ondes (43).

21. Appareil selon l'une ou plusieurs des revendications 12 à 20, **caractérisé en ce que** l'extrémité de la conduite d'amenée de gaz (7) est réalisée sous la forme d'une tuyère à deux ou plusieurs tubes (15), dont la/les chambre(s) annulaire(s) enveloppe (17) est/sont susceptibles(s) d'être raccordée(s) à une conduite amenant un hydrocarbure gazeux.

22. Appareil selon l'une ou plusieurs des revendications 12 à 21, **caractérisée en ce que** l'extrémité de la conduite d'amenée de gaz (7) est formée par une tuyère à plusieurs canaux, dont les ouvertures peuvent être raccordées à une ou plusieurs conduites d'amenée pour hydrocarbure, monoxyde de carbone, dioxyde de carbone, gaz inerte, vapeur, l'huile, eau, ou encore des mélanges de ceux-ci.

23. Appareil selon l'une ou plusieurs des revendications 12 à 22, **caractérisé en ce qu'**il est prévu un dispositif à rayonnement laser (29) dirigé vers l'ouverture de sortie (8) de la conduite d'amenée de gaz (7).

24. Appareil selon la revendication 23, **caractérisé en ce qu'**au dispositif à rayonnement laser (29) est associé un dispositif de focalisation.

25. Appareil selon l'une ou plusieurs des revendications 12 à 24, **caractérisé par** une conduite d'amenée de gaz (7) avec dispositif de diffusion d'ondes (42), qui plonge dans la masse en fusion (3).

26. Procédé pour le fonctionnement d'un appareil selon l'une ou plusieurs des revendications 12 à 25, **caractérisé en ce que**, pour la protection de la partie de l'appareil (7, 21, 23, 15) qui débouche dans le récipient (1), on régule l'admission d'un milieu protecteur, en augmentant par gradins ou en continu l'admission d'un milieu protecteur contenant un hydrocarbure lors d'une attaque croissante de la masse en fusion (3), c'est-à-dire lorsque la température augmente ou qu'il se produit une surchauffe de la masse en fusion (3).
